(19) Europäisches Patentamt
European Patent Office
Office européen des brevets

(11) Veröffentlichungsnummer: **0 300 272 B1**

(12) **EUROPÄISCHE PATENTSCHRIFT**

(45) Veröffentlichungstag der Patentschrift: **16.09.92**

(51) Int. Cl.[5]: **C07D 413/06**, C07D 413/14, C07D 471/10, A61K 31/445

(21) Anmeldenummer: **88110754.4**

(22) Anmeldetag: **06.07.88**

Die Akte enthält technische Angaben, die nach dem Eingang der Anmeldung eingereicht wurden und die nicht in dieser Patentschrift enthalten sind.

# (54) Oxazolidinone.

(30) Priorität: **18.07.87 DE 3723797**

(43) Veröffentlichungstag der Anmeldung:
**25.01.89 Patentblatt 89/04**

(45) Bekanntmachung des Hinweises auf die Patenterteilung:
**16.09.92 Patentblatt 92/38**

(84) Benannte Vertragsstaaten:
**AT BE CH DE ES FR GB IT LI NL SE**

(56) Entgegenhaltungen:
**DE-A- 1 951 273**
**US-A- 4 082 755**

**JOURNAL OF MEDICINAL CHEMISTRY, Band 13, Nr. 1, Januar 1970, Seiten 1-6, American Chemical Society; R.L. DUNCAN et al.: "Aroylpiperidines and pyrrolidines. A new class of potent central nervous system depressants"**

(73) Patentinhaber: **MERCK PATENT GESELLSCHAFT MIT BESCHRÄNKTER HAFTUNG**
**Frankfurter Strasse 250 Postfach 4119**
**W-6100 Darmstadt(DE)**

(72) Erfinder: **Prücher, Helmut**
**Königsbergerstrasse 9**
**W-6148 Heppenheim(DE)**
Erfinder: **Böttcher, Henning, Dr.**
**Soderstrasse 95**
**W-6100 Darmstadt(DE)**
Erfinder: **Gottschlich, Rudolf, Dr.**
**Buchenweg 1**
**W-6107 Reinheim(DE)**
Erfinder: **Minck, Klaus-Otto, Dr.**
**Büchestrasse 8**
**W-6105 Ober-Ramstadt(DE)**
Erfinder: **Haase, Anton, Dr.**
**Ahornweg 5**
**W-6109 Mühltal 1(DE)**
Erfinder: **Seyfried, Christoph, Dr.**
**Mathildenstrasse 6**
**W-6104 Jugenheim(DE)**

EP 0 300 272 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

**Beschreibung**

Die Erfindung betrifft neue Oxazolidinone der Formel I

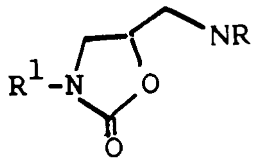

I

worin

R

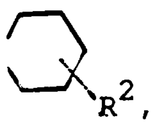

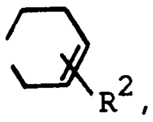

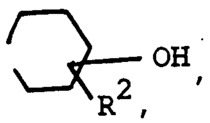

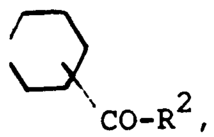

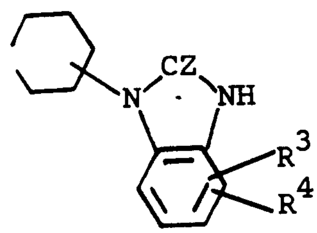

oder

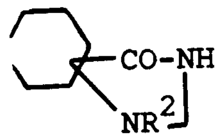

,

Z O oder S,

$R^1$ und $R^2$ jeweils unsubstituierte oder ein- oder zweifach durch Alkyl, Alkoxy, Alkylthio, Alkylsulfinyl und/oder Alkylsulfonyl mit jeweils 1-4 C-Atomen, Alkanoyloxy und/oder Alkanoylamino mit jeweils 1-6 C-Atomen, F, Cl, Br, OH und/oder $CF_3$ substituierte Phenyl-, oder Benzylreste,

$R^3$ und $R^4$ jeweils H, Alkyl oder Alkoxy mit jeweils 1-4 C-Atomen, F, Cl, Br, OH oder $CF_3$

bedeuten,

sowie ihre Salze.

Der Erfindung lag die Aufgabe zugrunde, neue Verbindungen aufzufinden, die zur Herstellung von Arzneimitteln verwendet werden können.

Es wurde gefunden, daß die genannten Substanzen bei guter Verträglichkeit wertvolle pharmakologische Eigenschaften besitzen. So zeigen sie z.B. das Zentralnervensystem beeinflussende, vorzugsweise dämpfende (z.B. sedierende, tranquillierende, neuroleptische und/oder antidepressive) Wirkungen. Im einzelnen haben die Verbindungen eine dämpfende Wirkung auf das Verhalten bei Mäusen (Methodik vgl. Irwin, Psychopharmacologia 13 (1968), 222-257), hemmen bei Mäusen das durch Apomorphin induzierte Kletterverhalten (Methodik vgl. Costall et al., European J. Pharmacol. 50 (1968), 39-50) oder induzieren contralaterales Drehverhalten in Hemiparkinson-Ratten (feststellbar nach der Methode von Ungerstedt et al., Brain Res. 24 (1970), 485-493), ohne daß nennenswerte kataleptische Nebenwirkungen auftreten (Methodik vgl. Dolini-Stola, Pharmakopsychiat. 6 (1973), 189-197). Weiterhin hemmen die Substanzen die Bindung von tritiierten Dopaminagonisten und -antagonisten an striäre Rezeptoren (feststellbar nach der Methode von Schwarcz et al., J. Neurochemistry 34 (1980), 772-778, und Creese et al., European J.Pharmacol. 46 (1977), 377-381). Zusätzlich hemmen die Verbindungen den Zungen-Kieferreflex bei der narkotisierten Ratte (feststellbar in Anlehnung an die Methoden von Barnett et al., European J.Pharmacol. 21 (1973), 178-182, und von Ilhan et al., European J.Pharmacol. 33 (1975), 61-64). Weiterhin treten analgetische und blutdrucksenkende Wirkungen auf; so wird der bei kathetertragenden wachen, spontan hypertonen Ratten (Stamm SHR/NIH-MO//CHB-EMD; Methode vgl. Weeks und Jones, Proc.Soc.Exptl.Biol.Med. 104 (1960), 646-648) direkt gemessene arterielle Blutdruck nach intragastraler Gabe der Verbindungen gesenkt.

Verbindungen der Formel I und ihre physiologisch unbedenklichen Säureadditionssalze können daher als Arzneimittelwirkstoffe und auch als Zwischenprodukte zur Herstellung anderer Arzneimittelwirkstoffe verwendet werden.

Verbindungen mit ähnlicher Struktur und/oder ähnlicher, jedoch schwächerer pharmakologischen Wirkung sind in DE-A-1951273 sowie in J. Med. Chem. 13, 1-6 (1970) beschrieben.

Gegenstand der Erfindung sind die Oxazolidinone der Formel I sowie ihre Salze.

Gegenstand der Erfindung ist ferner ein Verfahren zur Herstellung von Oxazolidinonen der Formel I sowie von deren Salzen, dadurch gekennzeichnet, daß man eine Verbindung der Formel II

$Ox-CH_2-X^1$ II

worin

Ox den Rest

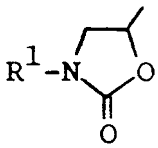

,

$X^1$ X oder $NH_2$,

X Hal, OH oder eine reaktionsfähig funktionell abgewandelte OH-Gruppe und

Hal Cl, Br oder J bedeuten und

$R^1$ die angegebene Bedeutung hat,

mit einer Verbindung der Formel III

$X^2-R-X^3$ III

worin

$X^2$ und $X^3$ gleich oder verschieden sind und, falls $X^1 = NH_2$ ist, jeweils X, andernfalls zusammen NH bedeuten und

R die angegebene Bedeutung hat,

umsetzt

und/oder daß man eine sonst der Formel I entsprechende Verbindung, die jedoch an Stelle eines oder mehrerer Wasserstoffatome eine oder mehrere reduzierbare Gruppen und/oder eine oder mehrere zusätzliche C-C- und/oder C-N-Bindungen enthält, mit einem reduzierenden Mittel behandelt

und/oder daß man zur Herstellung einer Verbindung der Formel I, worin

R 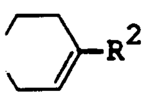 oder 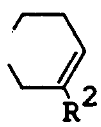

ist, eine Verbindung der Formel IV

$Ox-CH_2-R^5$ IV

worin

$R^5$ 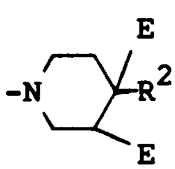 oder 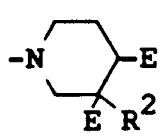 ,

der eine Rest E X, CN oder $NH_2$,
der andere Rest E H bedeutet und
Ox, $R^2$ und X die angegebenen Bedeutungen haben
mit einem HE-abspaltenden Mittel behandelt
oder daß man eine Verbindung der Formel V

$R^1$-NH-$CH_2$-CHOH-$CH_2$-NR V

worin R und $R^1$ die angegebenen Bedeutungen haben, mit einem reaktionsfähigen Derivat der Kohlensäure umsetzt
und/oder daß man gegebenenfalls in einer Verbindung der Formel I eine O-Alkylgruppe unter Bildung einer OH-Gruppe spaltet und/oder eine Verbindung der Formel I durch Reduktion in eine andere Verbindung der Formel I umwandelt
und/oder daß man eine Base der Formel I durch Behandeln mit einer Säure in eines ihrer Salze umwandelt.

Die Verbindungen der Formel I umschließen die folgenden Verbindungen (worin x 2-, 3- oder 4-Stellung, y 2-, 3-, 4-, 5- oder 6-Stellung und z 6-, 7- oder 8-Stellung bedeutet):
die 3-$R^1$-5-(x-$R^2$-piperidinomethyl)-oxazolidin-2-one (Ia),
die 3-$R^1$-5-(y-$R^2$ 1,2,3,6-tetrahydropyridinomethyl)oxazolidin-2-one (Ib),
die 3-$R^1$-5-(x-$R^2$-x-hydroxy-piperidinomethyl)-oxazolidin-2-one (Ic),
die 3-$R^1$-5-(x-$R^2$-CO-piperidinomethyl)-oxazolidin-2-one (Id),
die 3-$R^1$-5-[x-(2-oxobenzimidazolin-1-yl)-piperidinomethyl]oxazolidin-2-one, die in 4-, 5-, 6- und/oder 7-Stellung des Benzimidazolinrings durch $R^3$ und/oder $R^4$ substituiert sein können (Ie),
die 3-$R^1$-5-[x-(2-thioxobenzimidazolin-1-yl)-piperidinomethyloxazolidin-2-one, die in 4-, 5-, 6- und/oder 7-Stellung des Benzimidazolinrings durch $R^3$ und/oder $R^4$ substituiert sein können (If),
die 1-$R^2$-4-oxo-z-(3-$R^1$-oxazolidin-2-on-5-yl-methyl)-1,3,z-triazaspiro[4,5]-decane (Ig).

Bevorzugt sind Verbindungen der Formel Ic.

Im einzelnen handelt es sich um Verbindungen der
Formel Ia mit x = 2 (Iaa);
Formel Ia mit x = 3 (Iab);
Formel Ia mit x = 4 (Iac);
Formel Ib mit y = 2 (Iba);
Formel Ib mit y = 3 (Ibb);
Formel Ib mit y = 4 (Ibc);
Formel Ib mit y = 5 (Ibd);
Formel Ib mit y = 6 (Ibe);
Formel Ic mit x = 2 (Ica);
Formel Ic mit x = 3 (Icb);
Formel Ic mit x = 4 (Icc);
Formel Id mit x = 2 (Ida);
Formel Id mit x = 3 (Idb);
Formel Id mit x = 4 (Idc);
Formel Ie mit x = 2 (Iea);
Formel Ie mit x = 3 (Ieb);
Formel Ie mit x = 4 (Iec);
Formel If mit x = 2 (Ifa);
Formel If mit x = 3 (Ifb);
Formel If mit x = 4 (Ifc);
Formel Ig mit z = 6 (Iga);
Formel Ig mit z = 7 (Igb);
Formel Ig mit z = 8 (Igc).

Bevorzugt sind Verbindungen der Formel Icc, ferner solche der Formeln Iaa, Iab, Iac, Ibc, Ica, Icb, Idc, Iec, Ife und Igc.

In den Resten $R^1$ bis $R^4$ bedeutet Alkyl vorzugsweise Methyl, ferner auch Ethyl, n-Propyl, Isopropyl, n-Butyl, Isobutyl, sek.-Butyl oder tert.-Butyl. Alkoxy ist vorzugsweise Methoxy, ferner auch Ethoxy, n-Propoxy, Isopropoxy, n-Butoxy, Isobutoxy, sek.-Butoxy oder tert.-Butoxy.

Die Reste $R^1$ und $R^2$ sind bevorzugt unsubstituiertes oder einfach substituiertes Phenyl. Falls $R^1$ bzw. $R^2$ substituierte Phenylgruppen bedeuten, so können sie jedoch auch zweifach substituiert sein, wobei die Substituenten gleich oder verschieden sein können. Bevorzugte Substituenten an den Phenylgruppen sind

Methyl, Methoxy, F, Cl oder $CF_3$; ferner sind als Substituenten bevorzugt Ethyl, Ethoxy, Br und/oder OH. Im einzelnen sind $R^1$ und $R^2$ bevorzugt Phenyl, o-, m- oder p-Tolyl, o-, m- oder p-Methoxyphenyl, o-, m-oder p-Fluorphenyl, o-, m- oder p-Chlorphenyl, o-, m- oder p-Trifluormethylphenyl, ferner o-, m- oder p-Ethylphenyl, o-, m- oder p-Ethoxyphenyl, o-, m- oder p-Bromphenyl, o-, m- oder p-Hydroxyphenyl, 2,3-, 2,4- 2,5-, 2,6-, 3,4- oder 3,5-Dimethylphenyl, 2,3-, 2,4-, 2,5-, 2,6-, 3,4- oder 3,5-Dimethoxyphenyl, 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dihydroxyphenyl, 2-Methyl-4-chlorphenyl.

Der Rest $R^1$ ist ganz besonders bevorzugt p-Methoxyphenyl oder p-Fluorphenyl, der Rest $R^2$ Phenyl, p-Methoxyphenyl, p-Fluorphenyl, p-Chlorphenyl oder m-Trifluormethylphenyl.

Die Reste $R^3$ und $R^4$ können gleich oder verschieden sein. Sie bedeuten vorzugsweise beide H. Weiterhin ist bevorzugt der eine dieser Reste H, der andere F, Cl, OH oder $CF_3$.

Dementsprechend sind Gegenstand der Erfindung insbesondere diejenigen Verbindungen der Formeln I bzw. Ia bis Igc, in denen mindestens einer der genannten Reste eine der vorstehend angegebenen, insbesondere der vorstehend angegebenen bevorzugten Bedeutungen hat. Einige bevorzugte Gruppen von Verbindungen entsprechen den vorstehend angegebenen Formeln, worin die nicht näher bezeichneten Reste und Parameter die bei Formel I angegebene Bedeutung haben, worin jedoch

(a) $R^1$ Phenyl, Tolyl, Methoxyphenyl, Fluorphenyl, Chlorphenyl, Hydroxyphenyl, Trifluormethylphenyl oder Dimethoxyphenyl bedeutet;

(b) $R^1$ Phenyl, o-, m- oder p-Tolyl, m- oder p-Methoxyphenyl, p-Fluorphenyl, p-Chlorphenyl, m- oder p-Hydroxyphenyl, m-Trifluormethylphenyl oder 3,4-Dimethoxyphenyl bedeutet;

(c) $R^1$ p-Methoxyphenyl oder p-Fluorphenyl bedeutet;

(d) $R^2$ Phenyl, Tolyl, Methoxyphenyl, Fluorphenyl, Chlorphenyl oder Trifluormethylphenyl bedeutet;

(e) $R^2$ Phenyl, o-, m- oder p-Tolyl, p-Methoxyphenyl, p-Fluorphenyl, p-Chlorphenyl oder m-Trifluormethylphenyl bedeutet;

(f) $R^2$ Phenyl, p-Methoxyphenyl, p-Fluorphenyl, p-Chlorphenyl oder m-Trifluormethylphenyl bedeutet;

(g) $R^1$ Phenyl, o-, m- oder p-Tolyl, m- oder p-Methoxyphenyl, p-Fluorphenyl, p-Chlorphenyl, m- oder p-Hydroxyphenyl, m-Trifluormethylphenyl oder 3,4-Dimethoxyphenyl und
$R^2$ Phenyl, o-, m- oder p-Tolyl, p-Methoxyphenyl, p-Fluorphenyl, p-Chlorphenyl oder m-Trifluormethylphenyl bedeuten;

(h) $R^1$ p-Methoxyphenyl oder p-Fluorphenyl und
$R^2$ Phenyl, p-Methoxyphenyl, p-Fluorphenyl, p-Chlorphenyl oder m-Trifluormethylphenyl bedeuten.

Einige Verbindungen der Formel I können ein oder mehrere asymmetrische Kohlenstoffatome besitzen. Sie können daher als Racemate, falls mehrere asymmetrische Kohlenstoffatome vorhanden sind, auch als Gemische mehrerer Racemate sowie in verschiedenen optisch-aktiven Formen vorliegen.

Die Herstellung der Verbindungen der Formel I erfolgt im übrigen nach an sich bekannten Methoden, wie sie in der Literatur (z.B. in den Standardwerken wie Houben-Weyl, Methoden der Organischen Chemie, Georg-Thieme-Verlag, Stuttgart; Organic Reactions, John Wiley & Sons, Inc., New York) beschrieben sind, und zwar unter Reaktionsbedingungen, wie sie für die genannten Umsetzungen bekannt und geeignet sind. Dabei kann man auch von an sich bekannten, hier nicht näher erwähnten Varianten Gebrauch machen.

Die Ausgangsstoffe für das beanspruchte Verfahren können gewünschtenfalls auch in situ gebildet werden, derart, daß man sie aus dem Reaktionsgemisch nicht isoliert, sondern sofort weiter zu den Verbindungen der Formel I umsetzt.

In den Verbindungen der Formel II ist $X^1$ vorzugsweise X; dementsprechend sind in den Verbindungen der Formel III $X^2$ und $X^3$ vorzugsweise zusammen NH. Der Rest X ist vorzugsweise Cl oder Br; er kann jedoch auch J, OH oder eine reaktionsfähig funktionell abgewandelte OH-Gruppe bedeuten, insbesondere Alkylsulfonyloxy mit 1-6 (z.B. Methansulfonyloxy) oder Arylsulfonyloxy mit 6-10 C-Atomen (z.B. Benzolsulfonyloxy, p-Toluolsulfonyloxy, 1- oder 2-Naphthalin-sulfonyloxy).

Dementsprechend sind die Verbindungen der Formel I insbesondere durch Umsetzung von Verbindungen der Formeln $Ox\text{-}CH_2\text{-}Cl$, $Ox\text{-}CH_2\text{-}Br$ oder $Ox\text{-}CH_2\text{-}OSO_2CH_3$ mit Verbindungen der Formel III, worin $X^2$ und $X^3$ zusammen eine NH-Gruppe bedeuten (nachstehend als IIIa bezeichnet) erhältlich.

Die Verbindungen der Formeln II und III sind zum Teil bekannt; die nicht bekannten Verbindungen der Formeln II und III können leicht analog zu den bekannten Verbindungen herstellt werden. Primäre Alkohole der Formel $Ox\text{-}CH_2\text{-}OH$ sind z.B. durch Reduktion der entsprechenden Carbonsäuren oder ihrer Ester erhältlich. Behandeln mit Thionylchlorid, Bromwasserstoff, Phosphortribromid oder ähnlichen Halogenverbindungen liefert die entsprechenden Halogenide der Formel $Ox\text{-}CH_2\text{-}Hal$. Die entsprechenden Sulfonyloxyverbindungen sind erhältlich aus den Alkoholen $Ox\text{-}CH_2\text{-}OH$ durch Umsetzung mit den entsprechenden Sulfonsäurechloriden. Die Jodverbindungen der Formel $Ox\text{-}CH_2\text{-}J$ sind z.B. durch Einwirkung von Kaliumjodid auf die zugehörigen p-Toluolsulfonsäureester erhältlich. Die Amine der Formel Ox-$CH_2$-$NH_2$ sind z.B. aus den Halogeniden mit Phthalimidkalium oder durch Reduktion der entsprechenden Nitrile herstellbar.

Die Verbindungen der Formel IIIa sind teilweise bekannt (vgl. DE-OS 20 60 816) und z.B. erhältlich durch Umsetzung von 2-, 3- oder 4-Piperidon mit metallorganischen Verbindungen der Formel M-$R^2$ (worin M ein Li-Atom oder MgHal bedeutet), anschließende Hydrolyse zu den entsprechenden 2-$R^2$-2-hydroxy-, 3-$R^2$-3-hydroxy- oder 4-$R^2$-4-hydroxypiperidinen sowie, falls erwünscht, nachfolgende Dehydratisierung zu 2-, 3- oder 4-$R^2$-2,3-oder -3,4-dehydro-piperidinen und Hydrierung zu 2-, 3- oder 4-$R^2$-piperidinen. Verbindungen der Formel III ($X^2$ und $X^3$ = jeweils X) sind z.B. herstellbar durch Reduktion entsprechender Diester zu Diolen der Formel HO-R-OH (III, $X^2$ = $X^3$ = OH) und gegebenenfalls anschließende Umsetzung mit $SOCl_2$ bzw. $PBr_3$.

Die Umsetzung der Verbindungen II und III verläuft nach Methoden, wie sie für die Alkylierung von Aminen aus der Literatur bekannt sind. Man kann ohne Gegenwart eines Lösungsmittels die Komponenten miteinander verschmelzen, gegebenenfalls im geschlossenen Rohr oder im Autoklaven. Es ist aber auch möglich, die Verbindungen in Gegenwart eines indifferenten Lösungsmittels umzusetzen. Als Lösungsmittel eignen sich z.B. Kohlenwasserstoffe, wie Benzol, Toluol, Xylol; Ketone wie Aceton, Butanon; Alkohole wie Methanol, Ethanol, Isopropanol, n-Butanol; Ether wie Tetrahydrofuran (THF) oder Dioxan; Amide wie Dimethylformamid (DMF) oder N-Methyl-pyrrolidon; Nitrile wie Acetonitril, gegebenenfalls auch Gemische dieser Lösungsmittel untereinander oder Gemische mit Wasser. Der Zusatz eines säurebindenden Mittels, beispielsweise eines Alkali- oder Erdalkalimetallhydroxids, -carbonats oder -bicarbonats oder eines anderen Salzes einer schwachen Säure der Alkali- oder Erdalkalimetalle, vorzugsweise des Kaliums, Natriums oder Calciums, oder der Zusatz einer organischen Base wie Triethylamin, Dimethylanilin, Pyridin oder Chinolin oder eines Überschusses der Aminkomponente Ox-$CH_2$-$NH_2$ bzw. der Verbindung der Formel IIIa kann günstig sein. Die Reaktionszeit liegt je nach den angewendeten Bedingungen zwischen einigen Minuten und 14 Tagen, die Reaktionstemperatur zwischen etwa 0 und 150°, normalerweise zwischen 20 und 130°.

Es ist ferner möglich, eine Verbindung der Formel I zu erhalten, indem man ein Vorprodukt, das an Stelle von Wasserstoffatomen eine oder mehrere reduzierbare Gruppe(n) und/oder eine oder mehrere zusätzliche C-C- und/oder C-N-Bindung(en) enthält, mit einem reduzierenden Mittel behandelt, vorzugsweise bei Temperaturen zwischen -80 und +250° in Gegenwart mindestens eines inerten Lösungsmittels.

Reduzierbare (durch Wasserstoff ersetzbare) Gruppen sind insbesondere Sauerstoff in einer Carbonylgruppe, Hydroxyl, Arylsulfonyloxy (z.B.p-Toluolsulfonyloxy), N-Benzolsulfonyl, N-Benzyl oder O-Benzyl.

Es ist grundsätzlich möglich, Verbindungen, die nur eine, oder solche, die nebeneinander zwei oder mehr dieser Gruppen bzw. zusätzlichen Bindungen enthalten, reduktiv in eine Verbindung der Formel I überzuführen. Vorzugsweise bedient man sich hierzu der katalytischen Hydrierung, des nascierenden Wasserstoffs oder bestimmter komplexer Metallhydride wie $NaBH_4$.

Eine Gruppe bevorzugter Ausgangsstoffe für die Reduktion entspricht der Formel VI

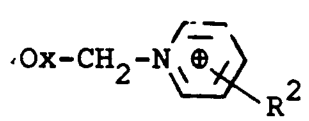 $An^{\ominus}$ VI

worin

Ox und $R^2$ die angegebenen Bedeutungen haben und $An^{\theta}$ ein Anion einer starken Säure, bevorzugt $Cl^{\theta}$ oder $Br^{\theta}$, bedeutet. Verbindungen der Formel VI sind z.B. herstellbar durch Umsetzung einer Verbindung der Formel 11 mit einem 2-, 3- oder 4-$R^2$-pyridin unter den Bedingungen, die oben für die Umsetzung von II und III angegeben sind.

Für die katalytische Hydrierung sind als Katalysatoren beispielsweise Edelmetall-, Nickel- und Kobaltkatalysatoren geeignet. Die Edelmetallkatalysatoren können auf Trägern (z.B. Platin oder Palladium auf Kohle, Palladium auf Calciumcarbonat oder Strontiumcarbonat), als Oxidkatalysatoren (z.B. Platinoxid), oder als feinteilige Metallkatalysatoren vorliegen. Nickel- und Kobaltkatalysatoren werden zweckmäßig als Raney-Metalle, Nickel auch auf Kieselgur oder Bimsstein als Träger eingesetzt. Die Hydrierung kann bei Raumtemperatur und Normaldruck oder auch bei erhöhter Temperatur und/oder erhöhtem Druck durchgeführt werden. Vorzugsweise arbeitet man bei Drucken zwischen 1 und 100 at und bei Temperaturen zwischen -80 und +150°, in erster Linie zwischen Raumtemperatur und +100°. Die Umsetzung wird zweckmäßig im sauren, neutralen oder basischen Bereich und in Gegenwart eines Lösungsmittels, wie Wasser, Methanol, Ethanol, Isopropanol, n-Butanol, Ethylacetat, Dioxan, Essigsäure oder THF durchgeführt;

man kann auch Gemische dieser Lösungsmittel untereinander anwenden.

Wird als Reduktionsmittel nascierender Wasserstoff verwendet, so kann man diesen z.B. durch Behandlung von Metallen mit schwachen Säuren oder mit Basen erzeugen. So kann man z.B. ein Gemisch von Zink mit Alkalilauge oder von Eisen mit Essigsäure verwenden. Geeignet ist auch die Verwendung von Natrium oder einem anderen Alkalimetall in einem Alkohol wie Ethanol, Isopropanol, Butanol, Amyl- oder Isoamylalkohol oder Phenol. Man kann ferner eine Aluminium-Nickel-Legierung in alkalischwäßriger Lösung, gegebenenfalls unter Zusatz von Ethanol verwenden. Auch Natrium- oder Aluminiumamalgam in wäßrigalkoholischer oder wäßriger Lösung sind zur Erzeugung des nascierenden Wasserstoffs geeignet. Die Umsetzung kann auch in heterogener Phase durchgeführt werden, wobei man zweckmäßig eine wäßrige und eine Benzol- oder Toluol-Phase verwendet.

Als Reduktionsmittel können ferner komplexe Metallhydride, wie $NaBH_4$, Diisobutylaluminiumhydrid oder $NaAl(OCH_2CH_2OCH_3)_2H_2$ sowie Diboran eingesetzt werden, falls erwünscht unter Zusatz von Katalysatoren wie $BF_3$, $AlCl_3$ oder LiBr. Als Lösungsmittel eignen sich hierfür insbesondere Ether wie Diethylether, Di-n-butylether, THF, Dioxan, Diglyme oder 1,2-Dimethoxyethan sowie Kohlenwasserstoffe wie Benzol. Für eine Reduktion mit $NaBH_4$ sind in erster Linie Alkohole wie Methanol oder Ethanol, ferner Wasser sowie wäßrige Alkohole als Lösungsmittel geeignet. Nach diesen Methoden reduziert man vorzugsweise bei Temperaturen zwischen -80 und +150°, insbesondere zwischen etwa 0 und etwa 100°.

Eine katalytische Hydrierung von Verbindungen der Formel VI liefert in der Regel die entsprechenden Piperidinderivate. Reduziert man dagegen die Verbindungen der Formel VI mit $NaBH_4$, so entstehen hauptsächlich die entsprechenden 1,2,3,6-Tetrahydropyridinderivate.

Man gelangt ferner zu Verbindungen der Formel I, worin NR eine 3- oder 4-$R^2$-1,2,3,6-tetrahydropyridylgruppe ist, indem man aus Verbindungen der Formel IV unter Ausbildung einer Doppelbindung HE abspaltet. Entsprechend der Definition von E kann es sich z.B. handeln um eine Abspaltung von Halogenwasserstoff, Wasser (Dehydratisierung), einer Carbonsäure oder einer anderen Säure, von Ammoniak oder von HCN. Die Ausgangsstoffe der Formel IV sind z.B. erhältlich durch Umsetzung von II ($X^1$ = X) mit einer Verbindung der Formel $HR^5$, worin $R^5$ die angegebene Bedeutung hat.

Falls einer der Reste E = Hal ist, kann dieser Substituent unter basischen Reaktionsbedingungen leicht eliminiert werden. Als Basen können verwendet werden: Alkalimetallhydroxide, Alkalimetallcarbonate, Alkoholate, wie z.B. Kalium-tert.-butylat, Amine, wie z.B. Dimethylanilin, Pyridin, Collidin oder Chinolin; als Lösungsmittel benutzt man z.B. Benzol, Toluol, Cyclohexan, THF oder tert.-Butanol. Die als Basen verwendeten Amine können auch im Überschuß als Lösungsmittel eingesetzt werden. Bedeutet der eine der Reste E eine OH-Gruppe, so benutzt man als wasserabspaltende Mittel vorzugsweise Säuren wie Essigsäure, Salzsäure oder Gemische beider. Der Zusatz eines Lösungsmittels (z.B. Wasser oder Ethanol) kann von Vorteil sein. Die Eliminierung von Acyl-, Alkylsulfonyl-sowie Alkoxysulfonyl-oxy- oder Amino-Resten kann unter ähnlichen Bedingungen durchgeführt werden. Eine Eliminierung von Sulfonsäure-Resten, z.B. die der Mesylate oder Tosylate, erfolgt schonend durch Kochen in DMF oder Dimethylsulfoxid mit Alkalimetallcarbonaten, z.B. $Li_2CO_3$, oder mit Kaliumacetat. Ammoniak kann bereits durch Erhitzen der Salze der entsprechenden Aminoverbindungen (insbesondere der 4-Aminoderivate) abgespalten werden.

In ähnlicher Weise kann HCN aus Verbindungen der Formel IV (eine Gruppe E = CN) durch Erhitzen abgespalten werden. Die Eliminierung von HE aus IV erfolgt allgemein bei Temperaturen zwischen 0 und etwa 250°, vorzugsweise zwischen 50 und 200°.

Verbindungen der Formel I sind ferner durch Umsetzung von Aminoalkoholen der Formel V mit reaktionsfähigen Derivaten der Kohlensäure erhältlich. Als solche eignen sich bevorzugt Dialkylcarbonate wie Dimethyl- oder Diethylcarbonat, Chlorameisensäureester wie Chlorameisensäure-methyl- oder -ethylester, N,N′-Carbonyldiimidazol oder Phosgen. Die Umsetzung gelingt zweckmäßig in Gegenwart eines inerten Lösungsmittels, vorzugsweise eines halogenierten Kohlenwasserstoffs wie Chloroform, eines Kohlenwasserstoffs wie Toluol oder eines Amids wie DMF bei Temperaturen zwischen etwa 20 und etwa 200, vorzugsweise zwischen 100 und 150°. Das Kohlensäurederivat wird zweckmäßig im Überschuß eingesetzt. Die Aminoalkohole V sind z.B. erhältlich durch Reaktion von Aminen der Formel $R^1$-$NH_2$ mit Epichlorhydrin zu ($R^1$-aminomethyl)-ethylenoxiden und nachfolgende Umsetzung mit Verbindungen der Formel IIIa.

Weiterhin kann man gegebenenfalls eine Verbindung der Formel I nach an sich bekannten Methoden in eine andere Verbindung der Formel I umwandeln.

So können Ether (O-Alkylderivate) gespalten werden, wobei die entsprechenden Hydroxyderivate entstehen. z.B. kann man die Ether spalten durch Behandeln mit Dimethylsulfid-Bortribromid-Komplex, z.B. in Toluol, 1,2-Dichlorethan, THF oder Dimethylsulfoxid, durch Verschmelzen mit Pyridin-oder Anilinhydrohalogeniden, vorzugsweise Pyridinhydrochlorid, bei etwa 150-250°, mit HBr/Essigsäure oder mit Al-trihalogeniden in chlorierten Kohlenwasserstoffen wie 1,2-Dichlorethan.

Eine erhaltene Base der Formel I kann mit einer Säure in das zugehörige Säureadditionssalz übergeführt werden. Für diese Umsetzung eignen sich bevorzugt Säuren, die physiologisch unbedenkliche Salze liefern. So können anorganische Säuren verwendet werden, z.B. Schwefelsäure, Halogenwasserstoffsäuren wie Chlorwasserstoffsäure oder Bromwasserstoffsäure, Phosphorsäuren wie Orthophosphorsäuren, Salpetersäure, Sulfaminsäure, ferner organische Säuren, im einzelnen aliphatische, alicyclische, araliphatische, aromatische oder heterocyclische ein- oder mehrbasige Carbon-, Sulfon- oder Schwefelsäuren, wie Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und disulfonsäuren, Laurylschwefelsäure. Säureadditionssalze, die nicht physiologisch unbedenklich sind (z.B. Pikrate), können sich zur Isolierung und Aufreinigung von Basen der Formel I eignen.

Die freien Basen der Formel I können, falls gewünscht, aus ihren Salzen durch Behandlung mit starken Basen wie Natrium- oder Kaliumhydroxid, Natrium- oder Kaliumcarbonat in Freiheit gesetzt werden.

Gegenstand der Erfindung ist ferner die Verwendung der Verbindungen der Formel I und ihrer physiologisch unbedenklichen Salze zur Herstellung pharmazeutischer Zubereitungen, insbesondere auf nicht-chemischem Wege. Hierbei können sie zusammen mit mindestens einem Träger-oder Hilfsstoff und gegebenenfalls in Kombination mit einem oder mehreren weiteren Wirkstoff(en) in eine geeignete Dosierungsform gebracht werden.

Gegenstand der Erfindung sind ferner Mittel, insbesondere pharmazeutische Zubereitungen, enthaltend mindestens eine Verbindung der Formel I und/oder eines ihrer physiologisch unbedenklichen Salze. Diese Zubereitungen können als Arzneimittel in der Human- oder Veterinärmedizin eingesetzt werden. Als Trägersubstanzen kommen organische oder anorganische Stoffe in Frage, die sich für die enterale (z.B. orale), parenterale oder topische Applikation eignen und mit den neuen Verbindungen nicht reagieren, beispielsweise Wasser, pflanzliche Öle, Benzylalkohole, Polyethylenglykole, Gelatine, Kohlehydrate wie Lactose oder Stärke, Magnesiumstearat, Talk, Vaseline. Zur enteralen Applikation dienen insbesondere Tabletten, Dragees, Kapseln, Sirupe, Säfte, Tropfen oder Suppositorien, zur parenteralen Applikation Lösungen, vorzugsweise ölige oder wäßrige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden.

Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z.B. ein oder mehrere Vitamine.

Die Verbindungen der Formel I und ihre physiologisch unbedenklichen Salze können bei der therapeutischen Behandlung des menschlichen oder tierischen Körpers und bei der Bekämpfung von Krankheiten verwendet werden, insbesondere von Schizophrenie und psychoreaktiven Störungen und Psychopathien, von Depressionen, von schweren chronischen Schmerzen und von Krankheiten, die mit erhöhtem Blutdruck einhergehen. Weiterhin können die Verbindungen bei der Behandlung extrapyramidaler Störungen Anwendung finden.

Dabei werden die erfindungsgemäßen Substanzen in der Regel in Analogie zu bekannten, im Handel befindlichen Präparaten (Thioridazin, Haloperidol) verabreicht, vorzugsweise in Dosierungen zwischen etwa 0,2 und 500 mg, insbesondere zwischen 0,2 und 50 mg pro Dosierungseinheit. Die tägliche Dosierung liegt vorzugsweise zwischen etwa 0,003 und 10 mg/kg Körpergewicht.

Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

In den nachstehenden Beispielen bedeutet "übliche Aufarbeitung": Man gibt, falls erforderlich, Wasser hinzu, extrahiert mit Dichlormethan, trennt ab, trocknet die organische Phase über Natriumsulfat, filtriert, dampft ein und reinigt durch Chromatographie an Kieselgel und/oder durch Kristallisation. Temperaturen sind in °C angegeben.

Beispiel 1

Man erhitzt 4,82 g 5-Methansulfonyloxymethyl-3-p-methoxyphenyl-oxazolidin-2-on [F. 126-128°; erhältlich durch Reaktion von p-Methoxyanilin mit Hydroxymethyl-oxiran zur 3-p-Methoxyanilino-1,2-propandiol,

Umsetzung mit Diethylcarbonat zu 3-p-Methoxyphenyl-5-hydroxymethyl-oxazolidon-2-on (F. 133-134°) und Reaktion mit $CH_3SO_2Cl$] mit 5,08 g 4-p-Chlorphenyl-4-hydroxy-piperidin in 25 ml DMF 1 Std. auf 95-100°, kühlt ab, arbeitet wie üblich auf und erhält 3-p-Methoxyphenyl-5-(4-p-chlorphenyl-4-hydroxy-piperidinomethyl)-oxazolidin-2-on, F. 143-144°.

Analog erhält man aus den entsprechenden 5-Hydroxymethyl3-$R^1$-oxazolidin-2-onen, z.B.:
5-Hydroxymethyl-3-p-fluorphenyl-oxazolidin-2-on (F. 107-108°)
5-Hydroxymethyl-3-p-chlorphenyl-oxazolidin-2-on (F. 113-115°)
5-Hydroxymethyl-3-m-trifluormethylphenyl-oxazolidin-2-on (F. 73-76°)
5-Hydroxymethyl-3-(3,4-dimethoxyphenyl)-oxazolidin-2-on (F. 153-154°)
über die entsprechenden 5-Methansulfonyloxymethyl-, 5-Chlormethyl- oder 5-Brommethyl-3-$R^1$-oxazolidin-2-one der Formel II, z.B.:
5-Chlormethyl-3-p-methoxyphenyl-oxazolidin-2-on (F. 98-100°)
5-Methansulfonyloxymethyl-3-p-fluorphenyl-oxazolidin-2-on (F. 136°)
5-Methansulfonyloxymethyl-3-p-chlorphenyl-oxazolidin-2-on (F. 123-125°)
5-Methansulfonyloxymethyl-3-m-trifluormethylphenyl-oxazolidin-2-on (F. 70-71°)
5-Methansulfonyloxymethyl-3-(3,4-dimethoxyphenyl)-oxazolidin-2-on (F. 147-149°)
mit den entsprechenden Piperidinderivaten der Formel III (worin $X^2$ und $X^3$ zusammen NH bedeuten):
3-Phenyl-5-(4-phenyl-piperidino-methyl)-oxazolidin-2-on, F. 122-123°
3-Phenyl-5-(2-benzyl-piperidino-methyl)-oxazolidin-2-on
3-m-Tolyl-5-(2-benzyl-piperidino-methyl)-oxazolidin-2-on
3-o-Methoxyphenyl-5-(2-benzyl-piperidino-methyl)-oxazolidin-2-on
3-m-Methoxyphenyl-5-(2-benzyl-piperidino-methyl)-oxazolidin-2-on
3-p-Methoxyphenyl-5-(2-benzyl-piperidino-methyl)-oxazolidin-2-on,2 Diastereomere, Hydrochloride, Zers. ab 200° und Zers. ab 205°
3-p-Fluorphenyl-5-(2-benzyl-piperidino-methyl)-oxazolidin-2-on, 2 Diastereomere, Hydrochloride, F. 207-208° und F. 209-210°
3-p-Chlorphenyl-5-(2-benzyl-piperidino-methyl)-oxazolidin-2-on
3-m-Trifluormethylphenyl-5-(2-benzyl-piperidino-methyl)-oxazolidin-2-on
3-(3,4-Dimethoxyphenyl)-5-(2-benzyl-piperidino-methyl)-oxazolidin-2-on
3-Phenyl-5-(3-benzyl-piperidino-methyl)-oxazolidin-2-on
3-m-Tolyl-5-(3-benzyl-piperidino-methyl)-oxazolidin-2-on
3-o-Methoxyphenyl-5-(3-benzyl-piperidino-methyl)-oxazolidin-2-on
3-m-Methoxyphenyl-5-(3-benzyl-piperidino-methyl)-oxazolidin-2-on
3-p-Methoxyphenyl-5-(3-benzyl-piperidino-methyl)-oxazolidin-2-on,Hydrochlorid-Hydrat, F. ab 139° (Zers.)
3-p-Fluorphenyl-5-(3-benzyl-piperidino-methyl)-oxazolidin-2-on, Hydrochlorid, F. 215-218° (Zers.)
3-p-Chlorphenyl-5-(3-benzyl-piperidino-methyl)-oxazolidin-2-on
3-m-Trifluormethylphenyl-5-(3-benzyl-piperidino-methyl)-oxazolidin-2-on
3-(3,4-Dimethoxyphenyl)-5-(3-benzyl-piperidino-methyl)-oxazolidin-2-on
3-Phenyl-5-(4-benzyl-piperidino-methyl)-oxazolidin-2-on
3-m-Tolyl-5-(4-benzyl-piperidino-methyl)-oxazolidin-2-on
3-o-Methoxyphenyl-5-(4-benzyl-piperidino-methyl)-oxazolidin-2-on
3-m-Methoxyphenyl-5-(4-benzyl-piperidino-methyl)-oxazolidin-2-on
3-p-Methoxyphenyl-5-(4-benzyl-piperidino-methyl)-oxazolidin-2-on,Hydrochlorid, F. ab 209° (Zers.)
3-p-Fluorphenyl-5-(4-benzyl-piperidino-methyl)-oxazolidin-2-on, Hydrochlorid, F. 224-225°
3-p-Chlorphenyl-5-(4-benzyl-piperidino-methyl)-oxazolidin-2-on
3-m-Trifluormethylphenyl-5-(4-benzyl-piperidino-methyl)-oxazolidin-2-on
3-(3,4-Dimethoxyphenyl)-5-(4-benzyl-piperidino-methyl)-oxazolidin-2-on
3-Phenyl-5-(4-hydroxy-4-phenyl-piperidino-methyl)-oxazolidin-2-on
3-m-Tolyl-5-(4-hydroxy-4-phenyl-piperidino-methyl)-oxazolidin-2-on, F. 121-122°
3-o-Methoxyphenyl-5-(4-hydroxy-4-phenyl-piperidino-methyl)-oxazolidin-2-on
3-m-Methoxyphenyl-5-(4-hydroxy-4-phenyl-piperidino-methyl)-oxazolidin-2-on
3-p-Methoxyphenyl-5-(4-hydroxy-4-phenyl-piperidino-methyl)-oxazolidin-2-on, F. 141-143°
3-p-Fluorphenyl-5-(4-hydroxy-4-phenyl-piperidino-methyl)-oxazolidin-2-on, F. 126-128°
3-p-Chlorphenyl-5-(4-hydroxy-4-phenyl-piperidino-methyl)-oxazolidin-2-on, F. 144-146°
3-m-Trifluormethylphenyl-5-(4-hydroxy-4-phenyl-piperidino-methyl)-oxazolidin-2-on, Hydrochlorid, F. 247-249° (Zers.)
3-(3,4-Dimethoxyphenyl)-5-(4-hydroxy-4-phenyl-piperidino-methyl)-oxazolidin-2-on
3-Phenyl-5-(4-hydroxy-4-p-methoxyphenyl-piperidino-methyl)-oxazolidin-2-on

3-m-Tolyl-5-(4-hydroxy-4-p-methoxyphenyl-piperidino-methyl)-oxazolidin-2-on, F. 121-122°
3-o-Methoxyphenyl-5-(4-hydroxy-4-p-methoxyphenyl-piperidino-methyl)-oxazolidin-2-on
3-m-Methoxyphenyl-5-(4-hydroxy-4-p-methoxyphenyl-piperidino-methyl)-oxazolidin-2-on
3-p-Methoxyphenyl-5-(4-hydroxy-4-p-methoxyphenyl-piperidino-methyl)-oxazolidin-2-on, F. 158-160°
3-p-Fluorphenyl-5-(4-hydroxy-4-p-methoxyphenyl-piperidino-methyl)-oxazolidin-2-on
3-p-Chlorphenyl-5-(4-hydroxy-4-p-methoxyphenyl-piperidino-methyl)-oxazolidin-2-on, F. 139-140°
3-m-Trifluormethylphenyl-5-(4-hydroxy-4-p-methoxyphenyl-piperidino-methyl)-oxazolidin-2-on
3-(3,4-Dimethoxyphenyl)-5-(4-hydroxy-4-p-methoxyphenyl-piperidino-methyl)-oxazolidin-2-on
3-Phenyl-5-(4-hydroxy-4-p-fluorphenyl-piperidino-methyl)-oxazolidin-2-on
3-m-Tolyl-5-(4-hydroxy-4-p-fluorphenyl-piperidino-methyl)-oxazolidin-2-on, F. 128-129°
3-o-Methoxyphenyl-5-(4-hydroxy-4-p-fluorphenyl-piperidino-methyl)-oxazolidin-2-on
3-m-Methoxyphenyl-5-(4-hydroxy-4-p-fluorphenyl-piperidino-methyl)-oxazolidin-2-on, F. 116-118°
3-p-Methoxyphenyl-5-(4-hydroxy-4-p-fluorphenyl-piperidino-methyl)-oxazolidin-2-on
3-p-Fluorphenyl-5-(4-hydroxy-4-p-fluorphenyl-piperidino-methyl)-oxazolidin-2-on
3-p-Chlorphenyl-5-(4-hydroxy-4-p-fluorphenyl-piperidino-methyl)-oxazolidin-2-on, F. 145-147°
3-m-Trifluormethylphenyl-5-(4-hydroxy-4-p-fluorphenyl-piperidino-methyl)-oxazolidin-2-on
3-(3,4-Dimethoxyphenyl)-5-(4-hydroxy-4-p-fluorphenyl-piperidino-methyl)-oxazolidin-2-on
3-Phenyl-5-(4-hydroxy-4-p-chlorphenyl-piperidino-methyl)-oxazolidin-2-on
3-m-Tolyl-5-(4-hydroxy-4-p-chlorphenyl-piperidino-methyl)-oxazolidin-2-on, F. 139-140°
3-o-Methoxyphenyl-5-(4-hydroxy-4-p-chlorphenyl-piperidino-methyl)-oxazolidin-2-on
3-m-Methoxyphenyl-5-(4-hydroxy-4-p-chlorphenyl-piperidino-methyl)-oxazolidin-2-on, F. 114-115°
3-p-Methoxyphenyl-5-(4-hydroxy-4-p-chlorphenyl-piperidino-methyl)-oxazolidin-2-on, F. 143-144°
3-p-Fluorphenyl-5-(4-hydroxy-4-p-chlorphenyl-piperidino-methyl)-oxazolidin-2-on, F. 134-136°
3-p-Chlorphenyl-5-(4-hydroxy-4-p-chlorphenyl-piperidino-methyl)-oxazolidin-2-on, F. 163-164°
3-m-Trifluormethylphenyl-5-(4-hydroxy-4-p-chlorphenyl-piperidino-methyl)-oxazolidin-2-on, Hydrochlorid, F. 235-237°
3-(3,4-Dimethoxyphenyl)-5-(4-hydroxy-4-p-chlorphenyl-piperidino-methyl)-oxazolidin-2-on, F. 123-125°
3-Phenyl-5-(4-hydroxy-4-m-trifluormethylphenyl-piperidino-methyl)-oxazolidin-2-on
3-m-Tolyl-5-(4-hydroxy-4-m-trifluormethylphenyl-piperidino-methyl)-oxazolidin-2-on, F. 136-137°
3-o-methoxyphenyl-5-(4-hydroxy-4-m-trifluormethylphenyl-piperidino-methyl)-oxazolidin-2-on
3-m-Methoxyphenyl-5-(4-hydroxy-4-m-trifluormethylphenyl-piperidino-methyl)-oxazolidin-2-on
3-p-Methoxyphenyl-5-(4-hydroxy-4-m-trifluormethylphenyl-piperidino-methyl)-oxazolidin-2-on, F. 137-138°
3-p-Fluorphenyl-5-(4-hydroxy-4-m-trifluormethylphenyl-piperidino-methyl)-oxazolidin-2-on, F. 147-148°
3-p-Chlorphenyl-5-(4-hydroxy-4-m-trifluormethylphenyl-piperidino-methyl)-oxazolidin-2-on
3-m-Trifluormethylphenyl-5-(4-hydroxy-4-m-trifluormethylphenyl-piperidino-methyl)-oxazolidin-2-on, F. 114-116°
3-(3,4-Dimethoxyphenyl)-5-(4-hydroxy-4-m-trifluormethylphenyl-piperidino-methyl)-oxazolidin-2-on
3-Phenyl-5-(4-benzyl-4-hydroxy-piperidino-methyl)-oxazolidin-2-on
3-Phenyl-5-(4-benzoyl-piperidino-methyl)-oxazolidin-2-on
3-m-Tolyl-5-(4-benzoyl-piperidino-methyl)-oxazolidin-2-on, F. 104-105°
3-o-Methoxyphenyl-5-(4-benzoyl-piperidino-methyl)-oxazolidin-2-on
3-m-Methoxyphenyl-5-(4-benzoyl-piperidino-methyl)-oxazolidin-2-on, Hydrochlorid, F. 261-263° (Zers.)
3-p-Methoxyphenyl-5-(4-benzoyl-piperidino-methyl)-oxazolidin-2-on, F. 130-132°
3-p-Fluorphenyl-5-(4-benzoyl-piperidino-methyl)-oxazolidin-2-on
3-p-Chlorphenyl-5-(4-benzoyl-piperidino-methyl)-oxazolidin-2-on
3-m-Trifluormethylphenyl-5-(4-benzoyl-piperidino-methyl)-oxazolidin-2-on
3-(3,4-Dimethoxyphenyl)-5-(4-benzoyl-piperidino-methyl)-oxazolidin-2-on, F. 131-133°
3-Phenyl-5-[4-(2-oxobenzimidazolin-1-yl)-piperidinomethyl]-oxazolidin-2-on
3-m-Tolyl-5-[4-(2-oxobenzimidazolin-1-yl)-piperidinomethyl]-oxazolidin-2-on, F. 210-212°
3-o-Methoxyphenyl-5-[4-(2-oxobenzimidazolin-1-yl)-piperidinomethyl]-oxazolidin-2-on
3-m-Methoxyphenyl-5-[4-(2-oxobenzimidazolin-1-yl)-piperidinomethyl]-oxazolidin-2-on, F. 168-170°
3-p-Methoxyphenyl-5-[4-(2-oxobenzimidazolin-1-yl)-piperidinomethyl-oxazolidin-2-on, F. 211-212°
3-p-Fluorphenyl-5-[4-(2-oxobenzimidazolin-1-yl)-piperidinomethyl]-oxazolidin-2-on, F. 216-217°
3-p-Chlorphenyl-5-[4-(2-oxobenzimidazolin-1-yl)-piperidinomethyl]-oxazolidin-2-on
3-m-Trifluormethylphenyl-5-[4-(2-oxobenzimidazolin-1-yl)-piperidinomethyl]-oxazolidin-2-on, F. 195-196°
3-(3,4-Dimethoxyphenyl)-5-[4-(2-oxobenzimidazolin-1-yl)-piperidinomethyl]-oxazolidin-2-on, F. 199-201°
3-Phenyl-5-[4-(2-thioxobenzimidazolin-1-yl)-piperidinomethyl]-oxazolidin-2-on
3-m-Tolyl-5-[4-(2-thioxobenzimidazolin-1-yl)-piperidinomethyl]-oxazolidin-2-on

3-o-Methoxyphenyl-5-[4-(2-thioxobenzimidazolin-1-yl)-piperidinomethyl]-oxazolidin-2-on
3-m-Methoxyphenyl-5-[4-(2-thioxobenzimidazolin-1-yl)-piperidinomethyl]-oxazolidin-2-on
3-p-Methoxyphenyl-5-[4-(2-thioxobenzimidazolin-1-yl)-piperidinomethyl]-oxazolidin-2-on
3-p-Fluorphenyl-5-[4-(2-thioxobenzimidazolin-1-yl)-piperidinomethyl]-oxazolidin-2-on
3-p-Chlorphenyl-5-[4-(2-thioxobenzimidazolin-1-yl)-piperidinomethyl]-oxazolidin-2-on
3-m-Trifluormethylphenyl-5-[4-(2-thioxobenzimidazolin-1-yl)-piperidinomethyl]-oxazolidin-2-on
3-(3,4-Dimethoxyphenyl)-5-[4-(2-thioxobenzimidazolin-1-yl)-piperidinomethyl]-oxazolidin-2-on
1-Phenyl-4-oxo-8-(3-phenyl-oxazolidin-2-on-5-yl-methyl)-1,3,8-triazaspiro[4,5]decan
1-Phenyl-4-oxo-8-(3-m-tolyl-oxazolidin-2-on-5-yl-methyl)-1,3,8-triazaspiro[4,5]decan, F. 166-168°
1-Phenyl-4-oxo-8-(3-o-methoxyphenyl-oxazolidin-2-on-5-yl-methyl)-1,3,8-triazaspiro[4,5]decan
1-Phenyl-4-oxo-8-(3-m-methoxyphenyl-oxazolidin-2-on-5-yl-methyl)-1,3,8-triazaspiro[4,5]decan, F. 157-158°
1-Phenyl-4-oxo-8-(3-p-methoxyphenyl-oxazolidin-2-on-5-yl-methyl)-1,3,8-triazaspiro[4,5]decan, F. 192-193°
1-Phenyl-4-oxo-8-(3-p-fluorphenyl-oxazolidin-2-on-5-yl-methyl)-1,3,8-triazaspiro[4,5]decan, F. 220-222°
1-Phenyl-4-oxo-8-(3-p-chlorphenyl-oxazolidin-2-on-5-yl-methyl)-1,3,8-triazaspiro[4,5]decan
1-Phenyl-4-oxo-8-(3-m-trifluormethylphenyl-oxazolidin-2-on-5-yl-methyl)-1,3,8-triazaspiro[4,5]decan, F. 188-190°
1-Phenyl-4-oxo-8-[3,4-dimethoxyphenyl)-oxazolidin-2-on-5-yl-methyl]-1,3,8-triazaspiro[4,5]decan, F. 192-194°

Beispiel 2

Ein Gemisch von 1,92 g 5-Aminomethyl-3-phenyl-oxazolidin-2-on [erhältlich durch Reaktion von Phenylisocyanat mit 1,3-Dichlor-2-propanol in Gegenwart von $AlCl_3$ zu N-Phenylcarbaminsäure-(1,3-Dichlor-2-propylester), Cyclisierung mit wässerigem KOH bei 100° zu 5-Chlormethyl-3-phenyl-oxazolidin-2-on, Reaktion mit Phthalimidkalium und nachfolgende Hydrolyse] und 2,15 g 1,5-Dichlor-3-phenyl-2-penten in 40 ml Aceton und 40 ml Wasser wird 24 Stunden gekocht und wie üblich aufgearbeitet. Man erhält 3-Phenyl-5-(4-phenyl-1,2,3,6-tetrahydropyridyl-1-methyl)-oxazolidin-2-on.

Beispiel 3

Zu einer Lösung von 4,41 g 1-(3-p-Methoxyphenyl-oxazolidin-2-on-5-yl-methyl)-4-phenyl-pyridiniumbromid (erhältlich aus 3-p-Methoxyphenyl-5-brommethyl-oxazolidin-2-on und 4-Phenylpyridin) in 50 ml 1 n NaOH gibt man unter Rühren 1 g $NaBH_4$ in 20 ml Wasser und rührt danach 3 Stunden bei 60°. Nach üblicher Aufarbeitung erhält man 3-p-Methoxyphenyl-5-(4-phenyl-1,2,3,6-tetrahydropyridyl-1-methyl)-oxazolidin-2-on.

Beispiel 4

Man erhitzt 1 g 3-p-Methoxyphenyl-5-(4-hydroxy-4-phenyl-piperidino-methyl)-oxazolidin-2-on mit 10 ml 1 n Salzsäure 2 Stunden auf 100°, arbeitet wie ublich auf und erhält 3-p-Methoxyphenyl-5-(4-phenyl-1,2,3,6-tetrahydropyridyl-1-methyl)-oxazolidin-2-on.

Beispiel 5

Ein Gemisch von 3,56 g 4-Hydroxy-1-(2-hydroxy-3-p-methoxy-anilino-propyl)-4-phenyl-piperidin (erhältlich durch Reaktion von p-Methoxyanilin mit 2,3-Epoxypropionsäureethylester zu 2-Hydroxy-3-p-methoxyanilino-propionsäureethylester, Reduktion mit $LiAlH_4$ zu 3-p-Methoxyanilino-1,2-propandiol, Dehydratisierung zum Epoxid und Umsetzung mit 4-Hydroxy-4-phenyl-piperidin), 1,5 g Diethylcarbonat und 50 ml DMF wird 4 Stunden auf 120° erhitzt. Nach Eindampfen und üblicher Aufarbeitung erhält man 3-p-Methoxyphenyl-5-(4-hydroxy-4-phenyl-piperidino-methyl)-oxazolidin-2-on, F. 141-143°.

Beispiel 6

Ein Gemisch von 10 g 3-p-Methoxyphenyl-5-(4-phenyl-1,2,3,6-tetrahydropyridyl-1-methyl)-oxazolidin-2-on und 10 g Pyridinhydrochlorid wird 3 Stunden bei 160° gerührt. Nach üblicher Aufarbeitung erhält man 3-p-Hydroxyphenyl-5-(4-phenyl-1,2,3,6-tetrahydropyridyl-1-methyl)-oxazolidin-2-on.

Beispiel 7

Man tropft eine Suspension von 3,8 g 3-p-Methoxyphenyl-5-(4-benzyl-piperidino-methyl)-oxazolidin-2-on in 50 ml 1,2-Dichlorethan zu einer siedenden Lösung von 15,6 g Dimethylsulfid-Bortribromid-Komplex in 50 ml 1,2-Dichlorethan, kocht noch 30 Minuten, arbeitet wie üblich auf und erhält 3-p-Hydroxyphenyl-5-(4-benzyl-piperidino-methyl)-oxazolidin-2-on.

Beispiel 8

Analog Beispiel 1 erhält man
3-p-Methoxyphenyl-5-(4-phenyl-piperidino-methyl)-oxazolidin-2-on,F. 154-156°
3-p-Fluorphenyl-5-(4-phenyl-piperidino-methyl)-oxazolidin-2-on, F. 143-145°
3-p-Hydroxyphenyl-5-(4-hydroxy-4-p-methoxyphenyl-piperidino-methyl)-oxazolidin-2-on, F. 136-138°
3-p-Hydroxyphenyl-5-(4-p-methoxyphenyl-piperidino-methyl)-oxazolidin-2-on, F. 137-138°
3-p-Methoxyphenyl-5-(4-hydroxy-4-p-trimethylacetoxyphenyl-piperidino-methyl)-oxazolidin-2-on, F. 170-172°
3-p-Methylthiophenyl-5-(4-hydroxy-4-p-methoxyphenyl-piperidino-methyl)-oxazolidin-2-on, F. 137-139°
3-p-Methylsulfinylphenyl-5-(4-hydroxy-4-p-methoxyphenyl-piperidino-methyl)-oxazolidin-2-on, F. 161-162°
3-p-Methylsulfonylphenyl-5-(4-hydroxy-4-p-methoxyphenyl-piperidino-methyl)-oxazolidin-2-on, F. 212-213°
3-p-Methoxyphenyl-5-(4-hydroxy-4-p-chlorphenyl-piperidino-methyl)-oxazolidin-2-on, F. 142° (Zers.)
3-p-Acetamidophenyl-5-(4-hydroxy-4-p-methoxyphenyl-piperidino-methyl)-oxazolidin-2-on, F. 235-237°
3-p-Acetamidophenyl-5-(4-hydroxy-4-p-chlorphenyl-piperidino-methyl)-oxazolidin-2-on, F. 230-232°.

Beispiel 9

Analog Beispiel 1 erhält man
3-p-Methoxyphenyl-5-(4-hydroxy-4-phenyl-piperidino-methyl)-oxazolidin-2-on,
S-(-)-Form, F. 154-155°; $[\alpha]_D^{20}$ -14,2° (Chloroform)
R-(+)-Form, F. 156-157°; $[\alpha]_D^{20}$ +14,6° (Chloroform)
3-p-Methoxyphenyl-5-(4-hydroxy-4-p-methoxyphenyl-piperidino-methyl)-oxazolidin-2-on,
S-(-)-Form, F. 136-138°; $[\alpha]_D^{20}$ -12,9° (Chloroform)
R-(+)-Form, F. 137-138°; $[\alpha]_D^{20}$ +13,7° (Chloroform)
3-p-Methoxyphenyl-5-(4-hydroxy-4-p-chlorphenyl-piperidino-methyl)-oxazolidin-2-on,
S-(-)-Form, F. 140-141°; $[\alpha]_D^{20}$ -13,9° (Chloroform)
R-(+)-Form, F. 140-141°; $[\alpha]_D^{20}$ +12,8° (Chloroform).

Die Ausgangsmaterialien sind wie folgt erhältlich:
R-(-)- bzw. S-(+)-1,2-O-Isopropylidenglycerin werden mit p-Toluolsulfonylchlorid/Pyridin zu den öligen p-Toluolsulfonaten und diese mit p-Methoxyanilin zu den öligen 2,2-Dimethyl-4-p-methoxyanilino-1,3-dioxolanen umgesetzt. Durch Säurehydrolyse erhält man daraus die beiden epimeren 1-p-Methoxyanilino-propan-2,3-diole (R-Form, F. 74-77°; $[\alpha]_D^{20}$ + 11,8° in Chloroform, S-Form, F. 79-80°; $[\alpha]_D^{20}$ -12,5° in Chloroform), die mit Diethylcarbonat/Na in die epimeren 3-p-Methoxyphenyl-5-hydroxymethyl-oxazolidin-2-one (R-Form, F. 164-165°; $[\alpha]_D^{20}$ -49,8 in DMSO; S-Form, F. 163-164°; $^{20}_D$ +51,4° in DMSO) und dann mit Methansulfonylchlorid/Pyridin in die entsprechenden Methansulfonate (R-Form, F. 149-150°; $[\alpha]_D^{20}$ -54,6° in DMSO; S-Form, F. 150-151°; $[\alpha]_D^{20}$ + 56,5° in DMSO) übergeführt werden.

Die nachstehenden Beispiele betreffen pharmazeutische Zubereitungen, die Amine der Formel I oder ihre Säureadditionssalze enthalten:

Beispiel A: Tabletten

Ein Gemisch von 1 kg 3-p-Methoxyphenyl-5-(4-hydroxy-4-p-chlorphenyl-piperidino-methyl)-oxazolidin-2-on, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

Beispiel B: Dragees

Analog Beispiel A werden Tabletten gepreßt, die anschließend in üblicher Weise mit einem Überzug aus Saccharose, Kartoffelstärke, Talk, Tragant und Farbstoff überzogen werden.

Beispiel C: Kapseln

2 kg 3-p-Methoxyphenyl-5-(4-hydroxy-4-phenyl-piperidino-methyl)-oxazolidin-2-on werden in üblicher Weise in Hartgelatinekapseln gefüllt, so daß jede Kapsel 20 mg des Wirkstoffs enthält.

Beispiel D: Ampullen

Eine Lösung von 1 kg 3-p-Fluorphenyl-5-(4-benzyl-piperidino-methyl)-oxazolidin-2-on-hydrochlorid in 60 1 zweifach destilliertem Wasser wird steril filtriert, in Ampullen abgefüllt, unter sterilen Bedingungen lyophilisiert und steril verschlossen. Jede Ampulle enthält 10 mg Wirkstoff.

Analog sind Tabletten, Dragees, Kapseln und Ampullen erhältlich, die einen oder mehrere der übrigen Wirkstoffe der Formel I und/oder ihre physiologisch unbedenklichen Säureadditionssalze enthalten.

**Patentansprüche**

**1.** Oxazolidinone der Formel I

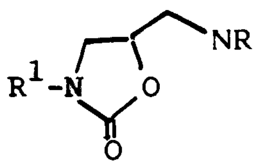

I

worin

R

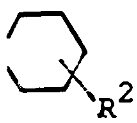 ,

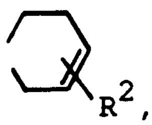 ,

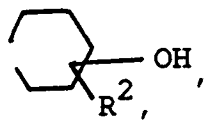 ,

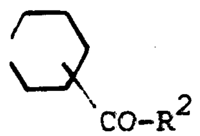 ,

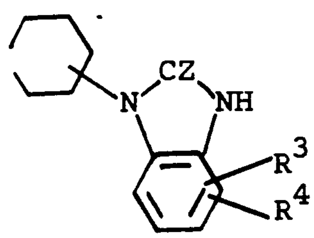

oder

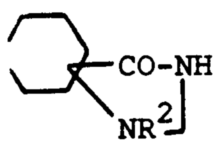 ,

| | |
|---|---|
| Z | O oder S, |
| $R^1$ und $R^2$ | jeweils unsubstituierte oder ein- oder zweifach durch Alkyl, Alkoxy, Alkylthio, Alkylsulfinyl oder Alkylsulfonyl mit jeweils 1-4 C-Atomen, Alkanoyloxy und/oder Alkanoylamino mit jeweils 1-6 C-Atomen, F, Cl, Br, OH und/oder $CF_3$ substituierte Phenyl- oder Benzyl- reste und |
| $R^3$ und $R^4$ | jeweils H, Alkyl oder Alkoxy mit jeweils 1-4 C-Atomen, F, Cl, Br, OH oder $CF_3$ |

bedeuten,
sowie ihre Salze.

**2.**

a) 3-p-Methoxyphenyl-5-(4-hydroxy-4-m-trifluormethylphenyl-piperidino-methyl)-oxazolidin-2-on;
b) 3-p-Fluorphenyl-5-(4-hydroxy-4-m-trifluormethylphenyl-piperidino-methyl)-oxazolidin-2-on;
c) 3-p-Fluorphenyl-5-(4-hydroxy-4-p-chlorphenylpiperidinomethyl)-oxazolidin-2-on;
d) 3-p-Methoxyphenyl-5-(4-hydroxy-4-p-chlorphenylpiperidinomethyl)-oxazolidin-2-on;
e) 3-p-Methoxyphenyl-5-(4-hydroxy-4-phenyl-piperidinomethyl)-oxazolidin-2-on;
f) 3-p-Methoxyphenyl-5-(4-hydroxy-4-p-methoxyphenyl-piperidinomethyl)-oxazolidin-2-on;

g) 3-p-Methoxyphenyl-5-(4-hydroxy-4-p-fluorphenylpiperidinomethyl)-oxazolidin-2-on.

**3.** Verfahren zur Herstellung von Oxazolidinonen der Formel I sowie von deren Salzen, dadurch gekennzeichnet, daß man eine Verbindung der Formel II

$Ox\text{-}CH_2\text{-}X^1$ II

worin

Ox den Rest

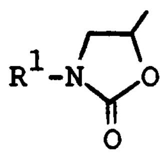 ,

$X^1$ X oder $NH_2$,

X Cl, Br, J, OH oder eine reaktionsfähig funktionell abgewandelte OH-Gruppe bedeuten und

$R^1$ die angegebene Bedeutung hat,

mit einer Verbindung der Formel III

$X^2\text{-}R\text{-}X^3$ III

worin

$X^2$ und $X^3$ gleich oder verschieden sind und, falls $X^1 = NH_2$ ist, jeweils X, andernfalls zusammen NH bedeuten und

R die angegebene Bedeutung hat,

umsetzt

und/oder daß man eine sonst der Formel I entsprechende Verbindung, die jedoch an Stelle eines oder mehrerer Wasserstoffatome eine oder mehrere reduzierbare Gruppen und/oder eine oder mehrere zusätzliche C-C- und/oder C-N-Bindungen enthält, mit einem reduzierenden Mittel behandelt

und/oder daß man zur Herstellung einer Verbindung der Formel I, worin

R 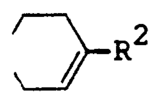 oder 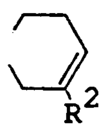

ist, eine Verbindung der Formel IV

$Ox\text{-}CH_2\text{-}R^5$ IV

worin

$R^5$ 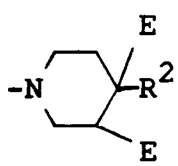 oder 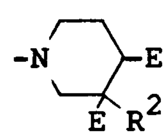 ,

der eine Rest E X, CN oder $NH_2$,

der andere Rest E H bedeutet und

Ox, $R^2$ und X die angegebenen Bedeutungen haben

mit einem HE-abspaltenden Mittel behandelt

oder daß man eine Verbindung der Formel V

$R^1$-NH-$CH_2$-CHOH-$CH_2$-NR V

worin R und $R^1$ die angegebenen Bedeutungen haben, mit einem reaktionsfähigen Derivat der Kohlensäure umsetzt
und/oder daß man gegebenenfalls in einer Verbindung der Formel I eine O-Alkylgruppe unter Bildung einer OH-Gruppe spaltet und/oder eine Verbindung der Formel I durch Reduktion in eine andere Verbindung der Formel I umwandelt
und/oder daß man eine Base der Formel I durch Behandeln mit einer Säure in eines ihrer Salze umwandelt.

**4.** Verfahren zur Herstellung pharmazeutischer Zubereitungen, dadurch gekennzeichnet, daß man eine Verbindung der Formel I und/oder eines ihrer physiologisch unbedenklichen Salze zusammen mit mindestens einem festen, flüssigen oder halbflüssigen Träger- oder Hilfsstoff und gegebenenfalls in Kombination mit einem oder mehreren weiteren Wirkstoff(en) in eine geeignete Dosierungsform bringt.

**5.** Pharmazeutische Zubereitung, gekennzeichnet durch einen Gehalt an mindestens einer Verbindung der Formel I und/oder einem ihrer physiologisch unbedenklichen Salze.

**6.** Verbindungen der Formel I und/oder ihre physiologisch unbedenklichen Salze zur Bekämpfung von Krankheiten.

**7.** Verwendung von Verbindungen der Formel I und/oder ihren physiologisch unbedenklichen Salzen bei der Bekämpfung von Krankheiten.

**8.** Verwendung von Verbindungen der Formel I und/oder ihren physiologisch unbedenklichen Salzen zur Herstellung von Arzneimitteln.

## Claims

**1.** Oxazolidinones of the formula I

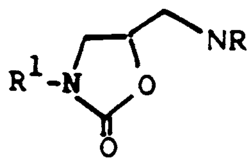

I

in which

R is 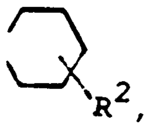 , 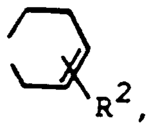 , 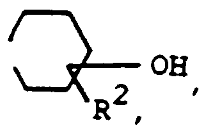 ,

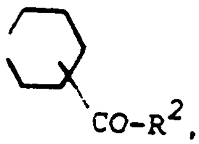 , 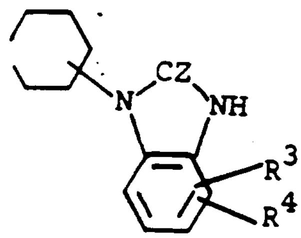

or 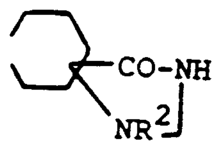 ,

Z is O or S,

$R^1$ and $R^2$ are each phenyl or benzyl radicals which are unsubstituted or mono- or disubstituted by alkyl, alkoxy, alkylthio, alkylsulfinyl or alkylsulfonyl each having 1-4 C atoms, alkanoyloxy and/or alkanoylamino each having 1-6 C atoms, F, Cl, Br, OH and/or $CF_3$ and

$R^3$ and $R^4$ are each H, alkyl or alkoxy having each 1-4 C atoms or are F, Cl, Br, OH or $CF_3$,

and also salts thereof.

**2.**

a) 3-p-methoxyphenyl-5-(4-hydroxy-4-m-trifluoromethyl-phenyl-piperidino-methyl)-oxazolidin-2-one;
b) 3-p-fluorophenyl-5-(4-hydroxy-4-m-trifluoromethyl-phenyl-piperidino-methyl)-oxazolidin-2-one;
c) 3-p-fluorophenyl-5-(4-hydroxy-4-p-chlorophenylpiperidinomethyl)-oxazolidin-2-one;
d) 3-p-methoxyphenyl-5-(4-hydroxy-4-p-chlorophenylpiperidinomethyl)-oxazolidin-2-one;
e) 3-p-methoxyphenyl-5-(4-hydroxy-4-phenyl-piperidinomethyl)-oxazolidin-2-one;
f) 3-p-methoxyphenyl-5-(4-hydroxy-4-p-methoxyphenylpiperidinomethyl)-oxazolidin-2-one;
g) 3-p-methoxyphenyl-5-(4-hydroxy-4-p-fluorophenylpiperidinomethyl)-oxazolidin-2-one.

**3.** Process for preparing oxazolidinones of the formula I and also of salts thereof, characterised in that a compound of the formula II

$Ox\text{-}CH_2\text{-}X^1$ II

in which

Ox is the radical

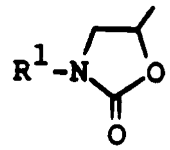,

$X^1$ is X or $NH_2$,
X is Cl, Br, I, Hal, OH or a reactive functionally modified OH group and
$R^1$ has the meaning given,

is reacted with a compound of the formula III

$X^2$-R-$X^3$ III

in which
$X^2$ and $X^3$ are identical or different and, if $X^1$ is $NH_2$, are each X, otherwise together they are NH and
R has the meaning given,

and/or that a compound which otherwise corresponds to the formula I but contains, instead of one or more hydrogen atoms, one or more reducible groups and/or one or more additional C-C and/or C-N bonds is treated with a reducing agent.
and/or that in order to prepare a compound of the formula I in which

R is 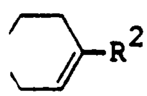 or 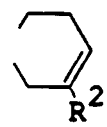,

a compound of the formula IV

Ox-$CH_2$-$R^5$ IV

in which

$R^5$ is 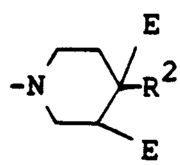 or 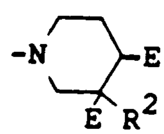,

one radical E is X, CN or $NH_2$,
the other radical E is H and
Ox, $R^2$ and X have the meanings given
is treated with an agent which eliminates HE, or that a compound of the formula V

$R^1$-NH-$CH_2$-CHOH-$CH_2$-NR V

in which R and $R^1$ have the meanings given are reacted with a reactive derivative of carbonic acid and/or that, if desired, in a compound of the formula I an O-alkyl group is cleaved to give an OH group and/or a compound of the formula I is converted by reduction to another compound of the formula I, and/or that a base of the formula I is converted to one of its salts by treatment with an acid.

**4.** Process for preparing pharmaceutical preparations, characterised in that a compound of the formula I and/or one of its physiologically safe salts is brought into a suitable dosage form together with at least one solid, liquid or semi-liquid carrier or auxiliary and, if desired, in combination with one or more further active substance(s).

**5.** Pharmaceutical preparation, characterised in that it contains at least one compound of the formula I and/or one of its physiologically safe salts.

**6.** Compounds of the formula I and/or their physiologically safe salts for fighting diseases.

**7.** Use of compounds of the formula I and/or their physiologically safe salts for fighting diseases.

**8.** Use of compounds of the formula I and/or their physiologically safe salts for preparing medicaments.

## Revendications

**1.** Oxazolidinone de formule I

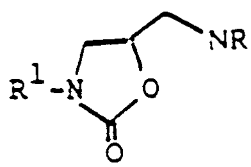

I

où

R représente

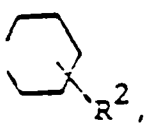

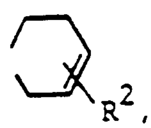

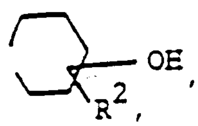

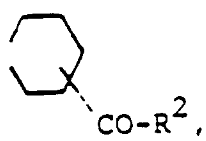

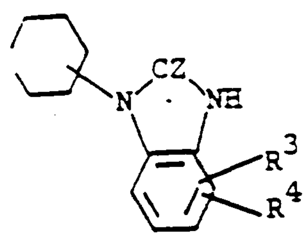

ou

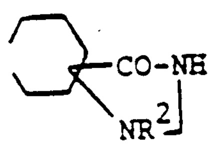

Z représente O ou S.

$R^1$ et $R^2$ représentent chaque fois des restes phényle ou benzyle non substitués ou substitués une ou deux fois par des alkyles, des alcoxy, des alkylthio, des alkylsulfinyles ou des alkylsulfonyles avec chaque fois de 1 à 4 atomes de C, des alcanoyloxy et/ou des alcanoylamino avec chaque fois de 1 à 6 atomes de C, par F, Cl, Br, OH et/ou $CF_3$, et

$R^3$ et $R^4$ représentent chacun H, un alkyle ou un alcoxy avec chaque fois de 1 à 4 atomes de C, F, Cl, Br, OH ou $CF_3$,

ainsi que leurs sels.

**2.**

a) 3-p-méthoxyphényl-5-(4-hydroxy-4-m-trifluorométhylphényl-pipéridino-méthyl)-oxazolidine-2-one;
b) 3-p-fluorophényl-5-(4-hydroxy-4-m-trifluorométhylphényl-pipéridino-méthyl)-oxazolidine-2-one;
c) 3-p-fluorophényl-5-(4-hydroxy-4-p-chlorophényl-pipéridino-méthyl)-oxazolidine-2-one;
d) 3-p-méthoxyphènyl-5-(4-hydroxy-4-p-chlorophényl-pipéridino-méthyl)-oxazolidine-2-one;
e) 3-p-méthoxyphényl-5-(4-hydroxy-4-phényl-pipéridinométhyl)-oxazolidine-2-one;
f) 3-p-méthoxyphényl-5-(4-hydroxy-4-p-méthoxyphénylpipéridinométhyl)-oxazolidine-2-one;
g) 3-p-méthoxyphényl-5-(4-hydroxy-4-p-fluorophénylpipéridinométhyl)-oxazolidine-2-one.

**3.** Procédé pour préparer des oxazolidinones de formule I ainsi que leurs sels, caractérisé en ce que l'on fait réagir un composé de formule II

$Ox\text{-}CH_2\text{-}X^1$ II

où

Ox représente le reste

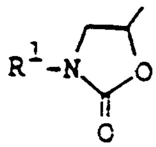

$X^1$ représente X ou $NH_2$,

X représente Cl, Br, J, OH ou un groupe OH fonctionnellement modifié pouvant réagir, et

$R^1$ a la signification donnée précédemment,

avec un composé de formule III

$X^2\text{-}R\text{-}X^3$ III

où

$X^2$ et $X^3$ sont identiques ou différents et représentent chacun X, si $X^1 = NH_2$, ou représentent ensemble NH, dans les autres cas et

R a la signification donnée précédemment,

et/ou en ce que l'on traite un composé correspondant à la formule I, par ailleurs, mais qui comporte, au lieu d'un ou plusieurs atomes d'hydrogène, un ou plusieurs groupes réductibles et/ou une ou plusieurs liaisons additionnelles C-C et/ou C-N, avec un moyen réducteur et/ou en ce que, pour préparer un composé de formule I, où

R est 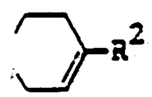 ou 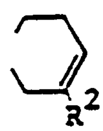 ,

on traite un composé de formule IV

$Ox\text{-}CH_2\text{-}R^5$ IV

où

$R^5$ est 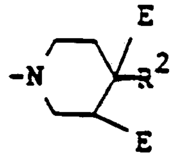 ou 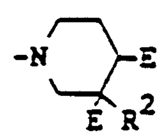 ,

l'un des restes E étant X, CN ou $NH_2$,

l'autre des restes E signifiant H, et

Ox, $R^2$ et X ayant les significations données plus haut

avec un moyen séparant HE

ou en ce que l'on fait réagir un composé de formule V

$R^1\text{-}NH\text{-}CH_2\text{-}CHOH\text{-}CH_2\text{-}NR$ V

où R et $R^1$ ont les significations données plus haut, avec un dérivé, susceptible de réagir, de l'acide carbonique
et/ou en ce que éventuellement dans un composé de formule I on scinde un groupe O-alkyle avec formation d'un groupe OH et/ou on transforme un composé de formule I, par réduction, en un autre composé de formule I
et/ou en ce que l'on transforme une base de formule I, par traitement avec un acide, en un de ses sels.

**4.** Procédé pour produire des préparations pharmaceutiques, caractérisé en ce que l'on met sous une forme de dosage appropriée un composé de formule I et/ou un de ses sels physiologiquement admissibles, avec au moins un support ou additif solide, liquide ou semi-liquide, et éventuellement en combinaison avec une ou plusieurs autres matières actives.

**5.** Préparation pharmaceutique, caractérisée en ce qu'elle contient au moins un composé de formule I et/ou un de ses sels physiologiquement admissibles.

**6.** Composés de formule I et/ou leurs sels physiologiquement admissibles pour lutter contre des maladies.

**7.** Utilisation de composés de formule I et/ou de leurs sels physiologiquement admissibles pour lutter contre des maladies.

**8.** Utilisation de composés de formule I et/ou de leurs sels physiologiquement admissibles pour préparer des médicaments.